# EUROPEAN PATENT SPECIFICATION

(11) **EP 3 189 774 B1**
(45) Date of publication and mention of the grant of the patent: **06.10.2021**
(21) Application number: 15837424.9
(22) Date of filing: 27.08.2015
(51) Int. Cl.: A61B 5/00, H04L 29/08

(54) **MEDICAL SENSOR AS WELL AS USING METHOD THEREFOR AND OPERATING DEVICE THEREOF**
MEDIZINISCHER SENSOR SOWIE VERWENDUNGSVERFAHREN DAFÜR UND BETRIEBSVORRICHTUNG DAFÜR
CAPTEUR MÉDICAL, SON PROCÉDÉ D'UTILISATION ET SON DISPOSITIF D'ACTIONNEMENT

(30) Priority: 03.09.2014 CN 201410446030
(43) Date of publication of application: 12.07.2017
(73) Proprietor: Medex (Beijing) Technology Limited Corporation, Beijing 100095 (CN)
(72) Inventor: GAO, Xiaofeng, Beijing 100095 (CN)
(74) Representative: Prinz & Partner mbB
(86) International application number: PCT/CN2015/088207
(87) International publication number: WO 2016/034064

(56) References cited:
- WO-A1-2013/142211
- WO-A2-2007/037949
- CN-A- 1 672 639
- CN-A- 103 083 829
- CN-A- 104 224 116
- CN-U- 204 218 879
- US-A1- 2007 208 233
- US-A1- 2011 131 320
- US-A1- 2013 036 412
- US-B1- 6 298 255
- "Information technology--Smart transducer interface for sensors and actuators--Part 7: Transducers to radio frequency identification (RFID) systems communication protocols and transducer electronic data sheet (TEDS) formats;ISO/IEC/IEEE 21451-7:2011(E) (Revision of 1451.7-2010", IEEE STANDARD, IEEE, PISCATAWAY, NJ, USA, 10 February 2012 (2012-02-10), pages 1-92, XP017694922, ISBN: 978-0-7381-6693-3
- None

## Description

### TECHNICAL FIELD

The present invention relates to the technical field of medical instrument. In particular, the present invention relates to a medical sensor, a method of using the same, and an operating device.

### RELATED ART

A medical sensor is a sensor applied in biomedical science for converting human physiological measurements into electrical information of definite functional relation with the physiological measurements information. Human physiological measurements include the two categories of electrical signals and non-electrical signals; or in terms of distribution, the categories of in-vivo measurements (various pressures such as blood pressure), body surface measurements (bioelectricity such as electrocardio measurements), and in vitro measurements (such as infrared and biomagnetics).

Along with the development of sensor and biomedical science, medical sensors are increasingly widely used in medical science. On one hand, medical sensors can be used for detection of normal or abnormal physiological parameters. For example, before a surgery on a patient of congenital heart disease, the endocardial pressure of the patient needs to be measured with a blood pressure sensor so as to estimate the degree of the defect. On the other hand, medical sensors can be used for continuously measuring certain physiological parameters to determine whether they are within a normal range. For example, monitoring instrument in an ICU should be equipped with a temperature sensor, a pulse sensor, a blood pressure sensor, a respiration sensor, and an electrocardiogram sensor, etc., so as to continuously monitor the temperature, pulse, blood pressure, respiration and electrocardiogram of critical patients and alarm the medical and nursing staff about critical status of the patients. Further, physiological procedures can be controlled based on the physiological parameters detected by a medical sensor. For example, when a synchronous respirator is used to rescue a patient, a respiration sensor is in need to detect respiration signal of the patient, thereby controlling the action of the respirator to be synchronized with human respiration.

At present, before the use of any medical sensor, the medical and nursing staffs need to know the model, technical specification and usage mode of a medical sensor, and initialize and set parameters for the medical sensor based on the information learned by themselves, so as to ensure that the medical sensor will work normally. In other words, the medical and nursing staff must learn the operation information of the medical sensor before use. This not only requires the learning capacity of the medical and nursing staff, but may severely affect the efficiency of medical diagnosis and treatment due to excessively long learning period.

Generic US 2007/208233 A1 shows a medical sensor with identification information that is identifiable by a particular electronic device and receives initialization parameters from the electronic device. An identification of the medical sensor is scanned to obtain identification information.

US 2011/131320 A1 shows a method for updating firmware of a sensor node through a gateway from a sensor storage server.

### SUMMARY

In view of this, the present invention intends to solve the following technical problem: how to realize immediate use when the medical sensor comes to hand, and eliminate the need for the medical and nursing staff to pre-study the usage of the medical sensor.

In order to solve the above technical problem the present invention provides a medical system comprising an operating device and a medical sensor, comprising the features of claim 1.

With regard to the above medical sensor, in a possible implementation, the configuration section is further configured to generate a startup message indicating that the medical sensor normally starts to work when the initialization and parameter setting are completed; and
the communication section is further configured to send the startup message to the electronic device.

With regard to the above medical sensor, in a possible implementation, in a possible implementation, the communication section is further configured to send the detection results of the medical sensor to the electronic device.

With regard to the above medical sensor, in a possible implementation, the identification includes one or more of a two-dimensional code, a bar code, a special appearance and an RFID label.

With regard to the above operating device, in a possible implementation, the processing section is further configured to:
based on the identification information, obtain service record of the medical sensor from the storage, wherein the service record includes one or more of usage count, accumulated service period, permitted usage count, permitted service period, permitted scope of use and service life;
based on the service record, determine whether or not the medical sensor is permitted to be used; and
when it is determined that the medical sensor is not permitted to be used, generate a deactivation message indicating that the medical sensor is forbidden to be used.

With regard to the above operating device, in a possible implementation, the communication section is further configured to:
receive a startup message from the medical sensor indicating that the medical sensor normally starts to work; and
the processing section is further configured to response to the startup message received by the communication section and update the service record of the medical sensor stored in the storage.

With regard to the above operating device, in a possible implementation, the processing section is further configured to:
add up time that has elapsed since the initialization parameters are sent; and
generate an error message indicating failure of the medical sensor when the time added up exceeds a predetermined time period yet the communication section still does not receive the startup message.

With regard to the above operating device, according to the invention, the processing section is further configured to:
based on the identification information, obtain diagnosis and treatment information associated with the medical sensor from the storage, wherein the diagnosis and treatment information includes one or more of name of hospital, name of department, and ID of medical and nursing staff; and the communication section is further configured to receive detection results of the medical sensor from the medical sensor; and
the processing section is further configured to respond to the detection results received by the communication section and send the detection results to a user terminal associated with the diagnosis and treatment information.

With regard to the above operating device, in a possible implementation, the storage comprises one or more of an internal data storage device built in the operating device, a portable data storage device attachable to the operating device and a remote data storage server capable of communicating with the operating device.

With regard to the above operating device, in a possible implementation, the scan section comprises one or more of a two-dimensional code reader, a bar code reader and an RFID label reader.

According to one embodiment, the present application further provides a usage of (a method of using) a medical sensor, comprising:
scanning identification of a medical sensor to obtain identification information of the medical sensor indicated by the identification;
based on the identification information, obtaining initialization parameters of the medical sensor from a storage, wherein the storage stores various data concerning medical sensors according to identification information; and
sending the initialization parameters to the medical sensor so that the medical sensor performs initialization and parameter setting based on the initialization parameters.

With regard to the above usage, in a possible implementation, before sending the initialization parameters to the medical sensor, the usage further comprises:
based on the identification information, obtaining service record of the medical sensor from the storage, wherein the service record includes one or more of usage count, accumulated service period, permitted usage count, permitted service period, permitted scope of use and service life;
based on the service record, determining whether or not the medical sensor is permitted to be used; and
when it is determined that the medical sensor is not permitted to be used, generating a deactivation message indicating that the medical sensor is forbidden to be used.

With regard to the above usage, in a possible implementation, after sending the initialization parameters to the medical sensor, the usage further comprises:
receiving a startup message from the medical sensor indicating that the medical sensor normally starts to work; and
in response to the startup message received, updating the service record of the medical sensor stored in the storage.

With regard to the above usage, in a possible implementation, after sending the initialization parameters to the medical sensor, the usage further comprises:
adding up time that has elapsed since the initialization parameters are sent; and
when the time added up exceeds a predetermined time period yet the startup message is still not received, generating an error message indicating failure of the medial sensor.

With regard to the above usage, according to one embodiment of the invention, after obtaining identification information of the medical sensor indicated by the identification, the usage further comprises:
based on the identification information, obtaining diagnosis and treatment information associated with the medical sensor from the storage, wherein the diagnosis and treatment information includes one or more of name of hospital, name of department, and ID of medical and nursing staff; and,
after sending the initialization parameters to the medical sensor, the usage further comprises:
   receiving detection results of the medical sensor from the medical sensor; and
   in response to the detection results received, sending the detection results to a user terminal associated with the diagnosis and treatment information.

The embodiment of the present disclosure provides a medical sensor with an identification indicating identification information of the medical sensor. The operating device can obtain the identification information of the medical sensor from the identification, and retrieve usage information of the medical sensor including the initialization parameters from a storage. Based on the usage information retrieved, the operating device automatically completes initialization and parameter setting of the medical sensor. Thus, a user can conveniently use the medical sensor without pre-studying the usage information of the medical sensor. This reduces burden of study and complexity in operation when using the medical sensor, and enhances working efficiency of medical diagnosis and treatment performed based on medical sensors.

Further features of the present invention will become apparent from the following description of exemplary embodiments with reference to the attached drawings.

### BRIEF DESCRIPTION OF THE DRAWINGS

The drawings enclosed in the description and forming a part of the description illustrates exemplary embodiments, features, and aspects of the present invention together with the description, and are used to interpret the principles of the present invention.
Fig. 1 is a schematic diagram of the structure of a medical system of the medical sensor and the operating device thereof according to one embodiment of the present invention;
Fig. 2 is a flow chart of a method of using a medical sensor;
Fig. 3 is a flow chart of a method of using the medical sensor;
Figs. 4a, 4b are flow charts of a method of using the medical sensor;
Fig. 5 is a flow chart of a method of using the medical sensor according to one other embodiment of the present invention;
Fig. 6 is a flow chart of a method of using the medical sensor according to one other embodiment of the present invention.

### DETAILED DESCRIPTION

Various exemplary embodiments, features, and aspects of the invention will be described in detail below with reference to the drawings. In the drawings, the same reference signs indicate elements with the same or similar functions. Though the drawings illustrate various aspects of the exemplary embodiments, unless it is pointed out particularly, it is unnecessary to draw the drawings according to the scale.

Herein the special word "exemplary" means "serving as an example, instance, or illustration". Any embodiment described in the follows as "exemplary" is not necessarily explained to be better than or preferred over other embodiments.

Further, in order to better describe the present invention, a number of specific details are described in the following embodiments. One skilled in the art should understand that the present invention can still be implemented without some specific details. In some embodiments, methods, means, elements, and circuits that are well known by one skilled in the art are not described in detail, so as to accentuate the subject of the present invention.

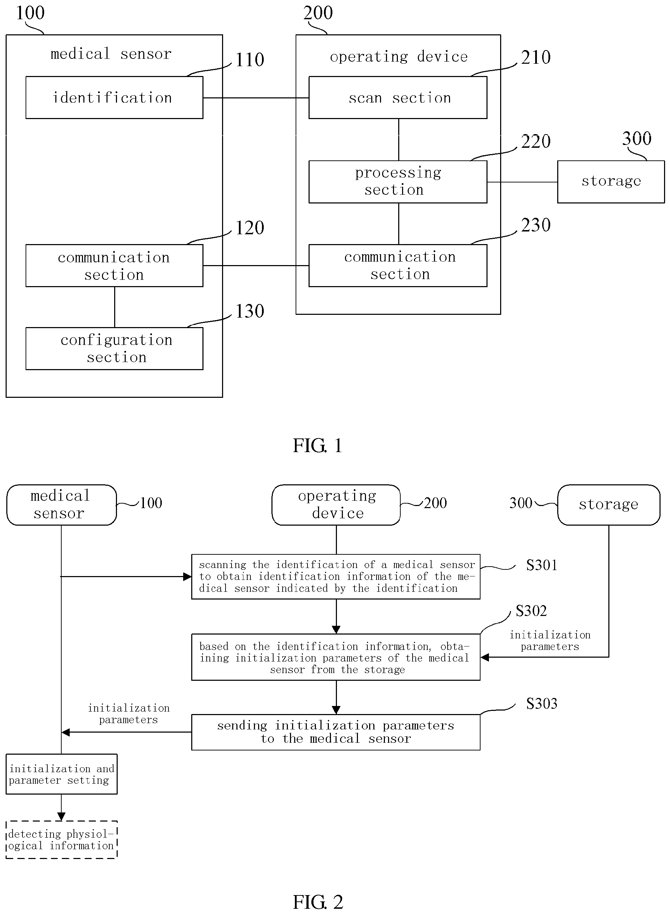

Fig. 1 is a schematic diagram of the structure of a medical system of the medical sensor and the operating device according to one embodiment of the present invention.

Referring to Fig. 1, the medical system mainly includes the medical sensor 100 and the operating device 200, wherein, the medical sensor 100 is mainly used for converting human physiological information into electrical information of certain functional relation with the physiological information; the operating device 200 is mainly used for communication with the medical sensor 100, so as to control one or more of initialization and parameter setting, startup and electrical information transmission of the medical sensor 100.

In addition, referring to Fig. 1, the medical system further comprises a storage 300 for storage of identification information of the medical sensor 100 and various data associated with the medical sensor 100. In one possible implementation, the storage 300 can be an internal data storage built in the operating device 200. In one other possible implementation, the storage 300 is a portable data storage attachable to the operating device 200, or a remote data storage capable of communicating with the operating device 200.

Before collection of human physiological information such as electrocardiogram, electroencephalogram, blood pressure, and blood oxygen using the medical sensor 100, the medical sensor 100 needs initialization and parameter setting for startup of the medical sensor 100. During the initialization and parameter setting of the medical sensor 100, initialization parameters of the medical sensor 100 can be obtained from the storage 300 via the operating device 200. Specifically, the operating device 200 can communicate with the medical sensor 100 to obtain identification information of the medical sensor 100, and search in the storage 300 according to the identification information to obtain usage information corresponding to the identification information, for example, the initialization parameters of the medical sensor 100. Further, the operating device 200 can send the retrieved initialization parameters to the medical sensor 100, and instruct the medical sensor 100 to automatically complete the initialization and parameter setting.

In one possible implementation, the medical sensor 100 can include identification 110, a communication section 120 and a configuration section 130, as shown in Fig. 1. The identification 110 is mainly used to carry identification information of the medical sensor 100, and can be identified by a particular electronic device, for example, the operating device 200. The communication section 120 is mainly used to receive from the electronic device the initialization parameters matching the identification information, in Fig. 1 specifically, the operating device 200. The configuration section 130 is connected to the communication section 120, mainly for completing the initialization and parameter setting of the medical sensor 100 according to the initialization parameters.

In one possible implementation, the operating device 200 can include a scan section 210, a processing section 220 and a communication section 230, as shown in Fig. 1. The scan section 210 is mainly used to scan the identification of the medical sensor 100 to obtain identification information of the medical sensor 100 indicated by the identification. The processing section 220 is connected to the scan section 210, mainly for obtaining initialization parameters of the medical sensor 100 from the storage 300 based on the identification information. The communication section 230 is connected to the processing section 220 and the medical sensor 100, mainly for sending the initialization parameters to the medical sensor 100, so that the medical sensor 100, specifically, the configuration section 130 of the medical sensor can perform initialization and parameter setting based on the initialization parameters.

In one possible implementation, the identification 110 can include any one or more of a two-dimensional code, a bar code, a special appearance and a Radio Frequency Identification (RFID) label. It should be noted that the identification 110 can be located on a surface of the medical sensor, for example, adhered to an outer surface of the case of the medical sensor 100, or be displayed on a display of the medical sensor 100. The special appearance can be a label adhered to the surface of the medical sensor, which is in a special shape easy to identify or recognize. In fact, the present invention does not limit the specific appearance of the identification 110. Any information carrier, as long as it stores identification information of the medical sensor 100 and can be identified by a particular electronic device, for example, the operating device 200, can be used as the identification 110 of the present invention. Correspondingly, any information obtaining device in existing technology that can retrieve the information carried by the identification 110 can be used as the scan section 210 of the operating device 200. In one possible implementation, the scan section 210 includes one or more of a two-dimensional code reader, a bar code reader, and an RFID label reader. For example, the identification 110 in form of a two-dimensional code utilizes the concept of "0" and "1" that constitutes internal logic basis of computers to indicate identification information of the medical sensor 100 with several geometric forms corresponding to the binary system; a two-dimensional code reader used as the scan section 210 can automatically obtain the identification information of the medical sensor 100 by scanning a two-dimensional code that is used as the identification 110.

In one possible implementation, the communication section 120 of the medical sensor 100 and the communication section 230 of the operating device 200 can communicate as two parties of the communication in a wired manner or in a wireless manner. In consideration of lowering the complexity of user's operation at best, the communication section 120 of the medical sensor 100 and the communication section 230 of the operating device 200 perform data interchange preferably via wireless technology such as WIFI and Bluetooth.

In one possible implementation, when the medical sensor 100 completes initialization and parameter setting via the configuration section 130, the configuration section 130 generates a startup message and sends the startup message through the communication section 120 to the operating device 200, so as to inform the operating device 200 on the status that the medical sensor starts to work normally.

After the operating device 200 sends initialization parameters to the medical sensor 100 through the communication section 230, due to some reason, for example, communication failure, the medical sensor 100 may not receive the initialization parameters; or due to physical failure of the medical sensor 100, the initialization and parameter setting may not be smoothly completed; thus, the medical sensor 100 cannot generate or send the startup message. Therefore, it is necessary that the operating device 200 monitors the status of initialization and parameter setting of the medical sensor 100. Specifically, in one possible implementation, the processing section 220 of the operating device 200 can be further configured to accumulate the time that has elapsed since the initialization parameters are sent, for example, by triggering a timer according to the sending of initialization parameters; and generate an error message indicating possible failure of the medical sensor 100 when the time accumulated exceeds a predetermined time period yet the communication section 230 still does not receive the startup message from the medical sensor 100. For example, assuming that the predetermined time period is 30 seconds, and when the time added up by the processing section 220 is 31 seconds, the communication section 230 still does not receive the startup message from the medical sensor 100, the processing section 220 will generate the above error message to instruct relative personnel to take measures in time.

In one possible implementation, after the medical sensor 100 normally works and collects detection results required for disease diagnosis, the medical sensor 100 can send the detection results to the operating device 200 through the communication section 120, so that the operating device 200 can display the detection results or perform further processing. Certainly, the operating device 200 can also send the detection results to a particular terminal for processing or display.

According to the invention, usage information associated with the medical sensor 100 stored in the storage 300 includes diagnosis and treatment information, such as name of hospital, name of department, and ID of medical and nursing staff, wherein, the diagnosis and treatment information can be set according to the type of the medical sensor 100 or according to the demand of the user of the medical sensor 100. Correspondingly, the operating device 200 can be configured to determine where to send detection results of the medical sensor 100 according to the diagnosis and treatment information. Specifically, the processing section 220 of the operating device 200 is configured to: based on identification information of the medical sensor 100, obtain diagnosis and treatment information associated with the medical sensor 100 from the storage 300, and when the communication section 230 receives detection results from the medical sensor 100, send the detection results to a user terminal associated with the diagnosis and treatment information.

For example, the medical sensor 100 is a sensor for detecting electroencephalogram, and the diagnosis and treatment information thereof shows that the name of hospital and department is "301 Hospital Brain Surgery Department". In that case, the operating device 200, e.g., specifically the processing section 220 therein, will send detection results of the medical sensor 100 to a terminal device of "301 Hospital Brain Surgery Department" which can be an information processing device such as a cell phone or a computer.

In one possible implementation, specifically, usage information associated with the medical sensor 100 which is stored according to the identification information in the storage 300 includes service record of the medical sensor 100, for example, one or more of usage count, accumulated service period, permitted usage count, permitted service period, permitted scope of use, and service life of the medical sensor. Thus, the processing section 220 of the operating device 200 can be further configured to: based on identification information of the medical sensor 100, obtain service record of the medical sensor 100 from the storage 300, and determine whether or not the medical sensor 100 is permitted to be used; and when it is determined that the medical sensor 100 is not permitted to be used, generate a deactivation message indicating that the medical sensor 100 is deactivated, so as to instruct that use of the medical sensor 100 is banned or forbidden.

For example, the usage count records the number of times the medical sensor 100 has been used. For some medical sensors being used by closely fitting to the body, in order to ensure hygiene, usually the total times of use can be limited. It may even be set so that the medical sensor can be used only once. In that case, the processing section 220 of the operating device 200 determines whether or not the medical sensor 100 is used for more than the permitted counts. If the medical sensor 100 is used for more than the permitted counts, a deactivation message is generated to ensure hygiene of the service of the medical sensor 100.

For another example, the accumulated service period records the total time in use of the medical sensor 100. In some cases, the medical sensor 100 may decrease in accuracy of detection after a certain period in use, for example, 10 days, where continuing use of the medical sensor may affect accuracy of the detection results. In that case, the processing section 220 of the operating device 200 determines whether the medical sensor 100 has exceeded a predetermined service period and generates a deactivation message after the predetermined service period is exceeded, so as to avoid effects brought thereby such as reduced accuracy of detection results.

In one possible implementation, the operating device 200 can be further configured that, after the communication section 230 receives the startup message from the medical sensor 100, the processing section 220 updates the service record stored in the storage 300. For example, the identification information of the medical sensor 100 is A; and the usage count stored in the storage 300 corresponding to the identification information A is "3". Thus, when the communication section 230 of the operating device 200 receives the startup message, the processing section 220 can perform updating process, i.e., to update the usage count stored in the storage 300 corresponding to the identification information A to "4".

This embodiment provides a medical sensor with an identification indicating identification information of the medical sensor. The operating device can obtain the identification information of the medical sensor through the identification and automatically complete initialization and parameter setting of the medical sensor based on the usage information retrieved. Thus, a user can conveniently use the medical sensor without pre-studying the usage information of the medical sensor. This reduces burden of study and complexity in operation in the use of the medical sensor, and enhances working efficiency of medical diagnosis and treatment performed based on medical sensors.

Fig. 2 is a flow chart of a method of using a medical sensor according to one embodiment of the present invention, which is mainly implemented by a user operating the operating device 200. The flow chart is described in detail with reference to the medical system shown by Fig. 1.

Referring to Fig. 2, a method of using the medical sensor according to one embodiment of the present invention mainly comprises:
Step S301, a user operating the operating device 200, for example pressing a particular button of the operating device 200 so that the operating device 200, specifically the scan section 210, scans identification 110 of the medical sensor 100 to obtain identification information of the medical sensor 100 indicated by the identification 110;
Step 302, the operating device 200, specifically the processing section 220 thereof, obtaining initialization parameters of the medical sensor 100 from storage 300 based on the identification information, wherein the storage 300 stores various data associated with the medical sensor according to identification information; and
Step S303, the operating device 200, specifically the communication section 230 thereof, sending the initialization parameters to the medical sensor 100, so that the medical sensor 100 performs initialization and parameter setting based on the initialization parameters.

As shown above, since the medical sensor 100 is provided with the identification 110, the operating device 200 can scan the identification to obtain the identification information of the medical sensor 100 and automatically commence the initialization and parameter setting of the medical sensor 100 based on the identification information obtained. According to the method of using the medical sensor in the embodiment of the present invention, by performing one simple operation on the operating device, for example, pressing a particular button, the user can use a professional medical sensor normally, which not only effectively reduces study burden and complexity in operation for the user, but effectively enhances efficiency in medical diagnosis and treatment work.

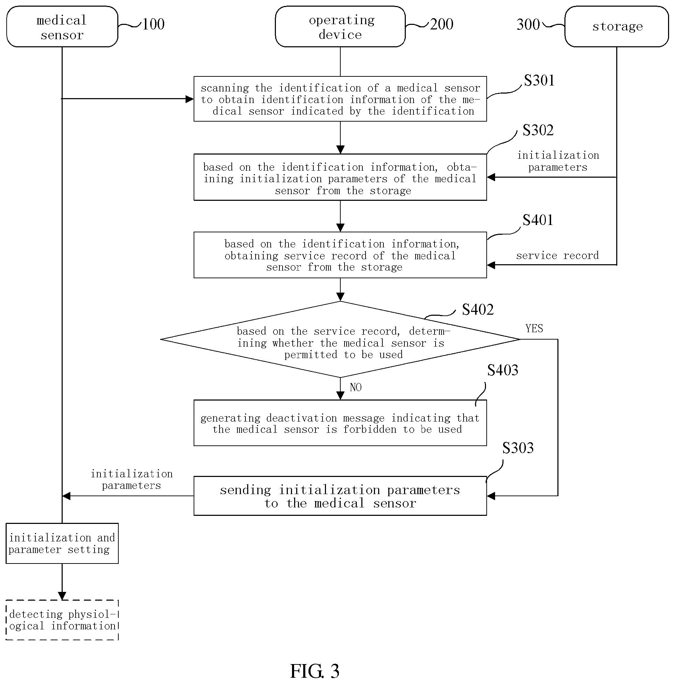

Fig. 3 is a flow chart of a method of using the medical sensor according to one other embodiment of the present invention. Referring to Fig. 3, the steps in Fig. 3 have the same function with the steps in Fig. 2 having the same reference signs. To be concise, detailed description for these steps is omitted.

As shown in Fig. 3, the usage is different from the usage of the medical sensor in the previous embodiment by that before step S303, the usage further comprises:
Step S401, the operating device 200, specifically the processing section 220 thereof, obtaining service record of the medical sensor 100 from the storage 300 based on identification information of the medical sensor 100 retrieved, wherein the service record includes one or more of usage count, accumulated service period, permitted usage count, permitted service period, permitted scope of use and service life of the medical sensor 100;
Step S402, the operating device 200, specifically the processing section 220 thereof, determining whether or not the medical sensor 100 is permitted to be used based on the service record retrieved, and executing the Step S303 if determined yes, otherwise executing the following Step S403;
Step S403, the operating device 200, specifically the processing section 220 thereof, generating a deactivation message indicating that the medical sensor 100 is deactivated when it is determined that the medical sensor 100 is not permitted to be used, to prompt the user to replace the medical sensor or take other measures.

Thus, since it can be determined whether or not the medical sensor is permitted to be used based on the service record of the medical sensor, the usage of the medical sensor according to the above embodiment of the present invention can effectively ensure safe use and accurate detection of the medical sensor. Specific examples can be referred to in the above description and will not be repeated here.

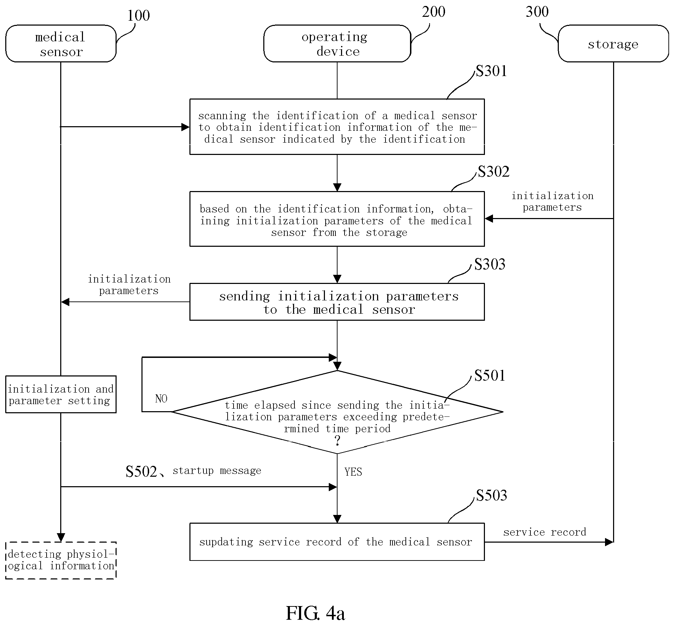

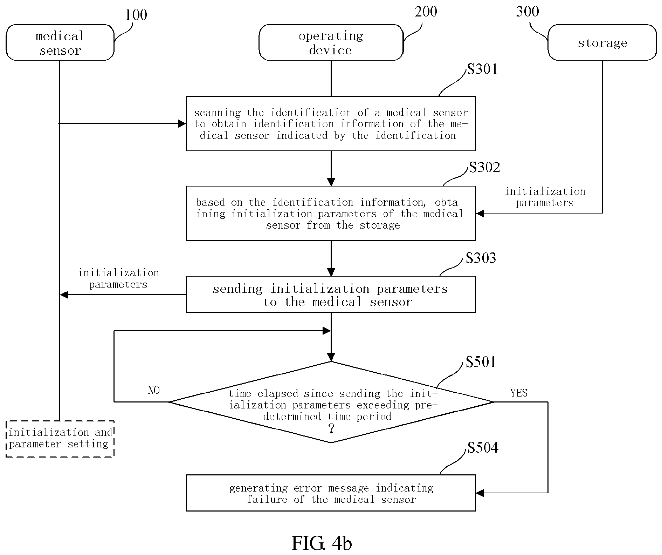

Figs. 4a and 4b are flow charts of methods of using the medical sensor according to one other embodiment of the present invention. Referring to Figs. 4a and 4b, the steps have the same function with the steps in Fig. 2 or Fig. 3 having the same reference signs. To be concise, detailed description for these steps is omitted.

As shown in Fig. 3, the usage is different from the usage of the medical sensor in the previous embodiments by: after step S303, the usage further comprising a Step S501. In Step S501, the operating device 200, specifically the processing section 220 thereof, continues to determine whether or not the time that has elapsed since the communication section 230 sends the initialization parameters to the medical sensor exceeds a predetermined time period, so as to detect failure of the medical sensor in time.

Specifically, in one possible implementation, after the medical sensor 100 smoothly completes initialization and parameter setting based on the initialization parameters received, the medical sensor 100 sends a startup message to the operating device 200 indicating that the medical sensor 100 starts to work normally. Correspondingly, on one hand, as shown in Fig. 4a, when the operating device 200, specifically the communication section 230 thereof, receives the startup message (Step S502) from the medical sensor 100 indicating that the medical sensor 100 starts to work normally, the operating device 200, specifically the processing section 220 stops executing Step S501 to execute Step S503 in response to the startup message to update service record of the medical sensor 100.

On the other hand, referring to Fig. 4b, if it is determined that the time elapsed since the communication section 230 sends the initialization parameters to the medical sensor 100 exceeds a predetermined time period, i.e., the determination of Step S501 is yes, yet the startup message from the medical sensor 100 is not received, the operating device 200, specifically the processing section 220 thereof, will generate an error message indicating that the medical sensor 100 may have a failure, so as to prompt relative personnel to take measures. Thus, when the medical sensor does not receive the initialization parameters due to communication failure or does not smoothly complete initialization and parameter setting due to physical failure of itself, the operating device 200 can be informed in time of the status that the medical sensor 100 fails to normally start working, avoiding endless waiting.

As shown above, after the operating device 100 sends the initialization parameters to the medical sensor 100, it can monitor the completion of the initialization and parameter setting of the medical sensor 100, and prompt the user to eliminate failure when the medical sensor 100 does not normally startup. This is very favorable for further enhancement in the efficiency of relative diagnosis and treatment work. Specific examples can be referred to in the above description and will not be repeated here.

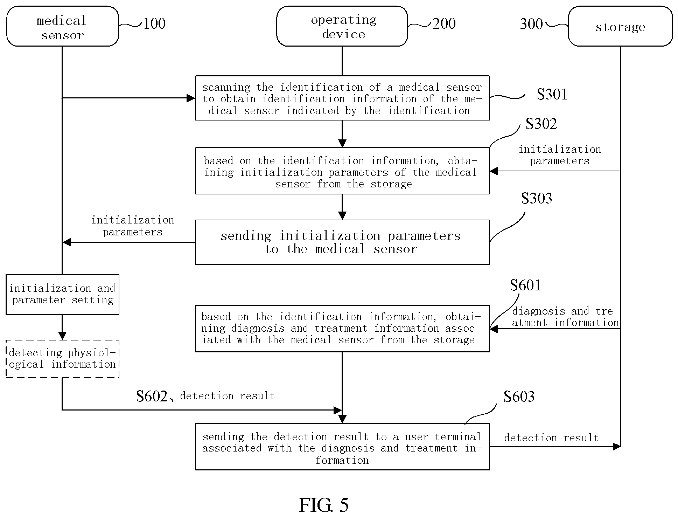

Fig. 5 is a flow chart of a method of using the medical sensor according to one other embodiment of the present invention. Referring to Fig. 5, the steps have the same function with the steps in Figs. 2, 3, 4a and 4b having the same reference signs. To be concise, detailed description for these steps is omitted. Referring to Fig. 5, the usage is different from the usage of the medical sensor in the previous embodiments by that after Step S301, the usage further comprises:
Step S601, the operating device 200, specifically the processing section 220, obtaining diagnosis and treatment information associated with the medical sensor 100 from the storage 300 based on the identification information, so as to determine where to send the detection results of the medical sensor 100 received, wherein the diagnosis and treatment information includes one or more of name of hospital, name of department, and ID of medical and nursing staff;
Step S602, the operating device 200, specifically the communication section 230, receiving the detection results of the medical sensor 100 from the medical sensor 100;
Step S603, the operating device 200, specifically the processing section 220, sending the detection results to a user terminal associated with the diagnosis and treatment information in response to the detection results received.

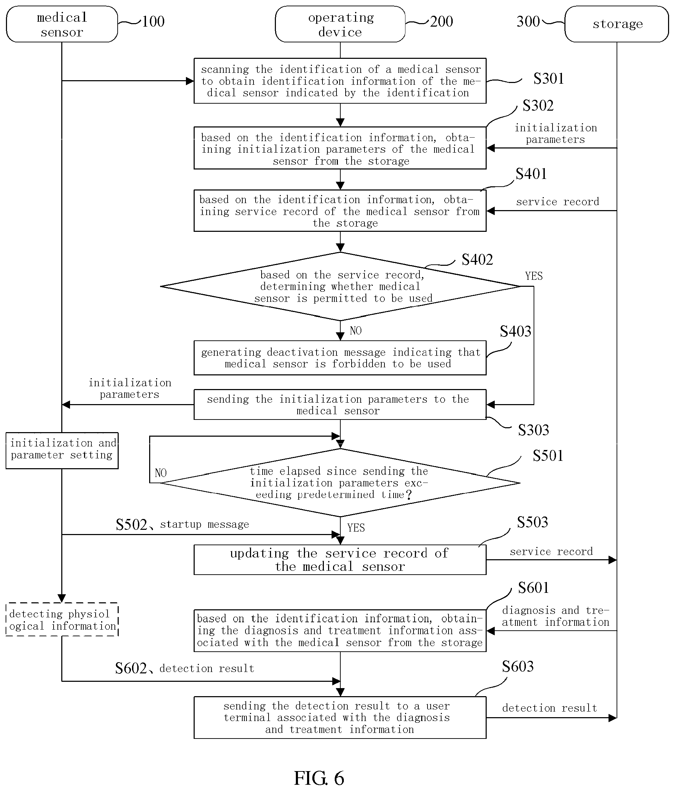

The usages and steps illustrated in Figs. 2-5 can be flexibly combined according to specific application. The present invention does not limit the combination of specific steps. For example, Fig. 6 is a flow chart of a method of using the medical sensor according to one other embodiment of the present invention combining the steps of the embodiments in Figs. 3, 4a and 5. It should be noted that although Fig. 6 illustrates to respectively execute Step S302 of retrieving initialization parameters, Step S401 of retrieving service record and Step S601 of retrieving diagnosis and treatment information, one skilled in the art should understand that the manner and time order of the execution of the above three steps are not limited to Fig. 6. For example, the three steps may be executed at one time, as long as the initialization parameters and the service record are retrieved before the initialization parameters are sent and the diagnosis and treatment information is retrieved before the detection results are sent.

The usage of medical sensor in this embodiment provides a medical sensor with identification indicating identification information of the medical sensor. The operating device can obtain the identification information of the medical sensor from the identification and retrieve usage information of the medical sensor from a storage based on the identification information. Based on the usage information retrieved, the operating device automatically completes initialization and parameter setting of the medical sensor. Thus, a user can conveniently use the medical sensor without pre-studying the usage information of the medical sensor. This reduces study burden and complexity in operation in the use of the medical sensor and enhances working efficiency of medical diagnosis and treatment performed based on medical sensors.

The foregoing description are specific illustrative embodiments of the present invention. However, the present invention is not limited to the disclosed exemplary embodiments. Any modification and substitution conceived by one skilled in the art within the technical scope of the disclosure of the present invention should be covered by the protection scope of the present invention. Therefore, the protection scope of the present invention should be accorded the scope of the following claims.

## Claims

1. A medical system comprising a medical sensor (100) and an operating device (200),
the medical sensor (100) comprising:
an identification (110) that indicates identification information of the medical sensor (100) and that is identifiable by the operating device (200), wherein the identification (110) includes one or more of a two-dimensional code, a bar code, a special appearance, and an RFID label; and
a first communication section (120) for receiving initialization parameters matching the identified identification information from the operating device (200); and
the operating device (200) comprising:
a scan section (210) for scanning the identification (110) included with the medical sensor (100) to obtain identification information of the medical sensor (100) indicated by the identification (110), the scan section (210) including one or more of a two-dimensional code reader, a bar code reader, and an RFID label reader;
a processing section (220) for obtaining initialization parameters of the medical sensor (100) from a storage (300) based on the identification information, wherein the storage (300) stores various data associated with the medical sensor (100) according to the identification information;
the medical sensor (100) further comprising: a configuration section (130) that is connected with the first communication section (120), for automatically completing initialization and parameter setting of the medical sensor (100) according to the initialization parameters; and
the operating device (200) further comprises: a second communication section (230) for sending the initialization parameters to the medical sensor (100), and instructing the medical sensor (100) to automatically perform initialization and parameter setting based on the initialization parameters,
the processing section (220) further being configured to obtain diagnosis and treatment information associated with the medical sensor (100) from the storage (300) based on the identification information, the diagnosis and treatment information including one or more of name of hospital, name of department, and ID of medical and nursing staff and
the second communication section (230) further being configured to receive detection results of the medical sensor (100) from the medical sensor (100); and
the processing section (220) further being configured to send the detection results to a user terminal associated with diagnosis and treatment information in response to the detection results received by the second communication section (230).

2. The medical system according to claim 1, wherein
the configuration section (130) is further configured to generate a startup message indicating that the medical sensor (100) starts to work normally when the initialization and parameter setting are completed; and
the first communication section (120) is further configured to send the startup message to the operating device (200).

3. The medical system according to claim 1, wherein
the first communication section (120) is further configured to send detection results of the medical sensor (100) to the operating device (200).

4. The medical system according to claim 1, wherein the processing section (220) being further configured to
based on the identification information, obtain service record of the medical sensor (100) from the storage (300), the service record including one or more of usage count, accumulated service period, permitted usage count, permitted service period, permitted scope of use, and service life of the medical sensor (100);
based on the service record, determine whether or not the medical sensor (100) is permitted to be used; and
when it is determined that the medical sensor (100) is not permitted to be used, generate a deactivation message indicating that the medical sensor (100) is forbidden to be used.

5. The medical system according to claim 4, wherein
the second communication section (230) is further configured to receive from the medical sensor (100) a startup message indicating that the medical sensor (100) starts to work normally; and
the processing section (220) is further configured to update the service record of the medical sensor (100) stored in the storage (300) in response to the startup message received by the second communication section (230).

6. The medical system according to claim 5, wherein the processing section (220) is further configured to
accumulate time that has elapsed since the initialization parameter is sent; and
when the time accumulated exceeds a predetermined time period yet the second communication section (230) still does not receive the startup message, generate an error message indicating failure of the medical sensor (100).

7. The medical system according to one of claims 1 to 6, wherein the storage (300) includes one or more of an internal data storage device built in the operating device (200), a portable data storage device attachable to the operating device (200), and a remote data storage server capable of communicating with the operating device (200).

8. A method of using a medical sensor of a medical system according to any of the preceding claims, comprising:
scanning an identification of the medical sensor to obtain identification information included with the medical sensor indicated by the identification (S301);
based on the identification information, obtaining initialization parameters of the medical sensor from a storage, wherein the storage stores various data associated with the medical sensor according to identification information (S302);
causing the initialization parameters to be sent to the medical sensor (S303), and
instructing the medical sensor to automatically perform initialization and parameter setting based, on the initialization parameters, and
after obtaining the identification information of the medical sensor indicated by the identification, further, based on the identification information, obtaining diagnosis and treatment information associated with the medical sensor from the storage (S601), wherein the diagnosis and treatment information includes one or more of name of hospital, name of department and ID of medical and nursing staff, and,
after sending the initialization parameters to the medical sensor, further receiving detection results of the medical sensor from the medical sensor (S602); and
in response to receiving the detection results, sending the detection results to a user terminal associated with the diagnosis and treatment information (S603).

9. The method according to claim 8, before sending the initialization parameters to the medical sensor, further comprising:
based on the identification information, obtaining service record of the medical sensor from the storage (S401), wherein the service record includes one or more of usage count, accumulated service period, permitted usage count, permitted service period, permitted scope of use, and service life of the medical sensor;
based on the service record, determining whether or not the medical sensor is permitted to be used (S402); and
when it is determined that the medical sensor is not permitted to be used, generating a deactivation message that indicates the medical sensor is forbidden to be used (S403).

10. The method according to claim 9, after sending the initialization parameters to the medical sensor, further comprising:
receiving a startup message from the medical sensor indicating that the medical sensor starts to work normally (S502); and
in response to the received startup message, updating the service record of the medical sensor stored in the storage (S503).

11. The method according to claim 10, after sending the initialization parameters to the medical sensor, further comprising:
accumulating time that has elapsed since the initialization parameter is sent (S501); and
when the time accumulated exceeds a predetermined time period yet the startup message is still not received, generating an error message indicating failure of the medical sensor (S504).

## Patentansprüche

1. Medizinisches System mit einem medizinischen Sensor (100) und einer Bedienvorrichtung (200),
wobei der medizinische Sensor (100) Folgendes umfasst:
eine Kennung (110), die Kennungsinformationen des medizinischen Sensors (100) angibt und die von der Bedienvorrichtung (200) erkannt werden kann, wobei die Kennung (110) einen zweidimensionalen Code und/oder einen Strichcode und/oder ein besonderes Erscheinungsbild und/oder ein RFID-Etikett aufweist, und
einen ersten Kommunikationsabschnitt (120) zum Empfangen von Initialisierungsparametern, die zu den erkannten Kennungsinformationen passen, von der Bedienvorrichtung (200), und
wobei die Bedienvorrichtung (200) Folgendes umfasst:
einen Abtastabschnitt (210) zum Abtasten der Kennung (110), die in dem medizinischen Sensor (100) enthalten ist, um durch die Kennung (110) angegebene Kennungsinformationen des medizinischen Sensors (100) zu erhalten, wobei der Abtastabschnitt (210) einen Leser für zweidimensionale Codes und/oder einen Strichcodeleser und/oder einen RFID-Etikettenleser aufweist,
einen Verarbeitungsabschnitt (220) zum Erhalten von Initialisierungsparametern des medizinischen Sensors (100) aus einem Speicher (300) anhand der Kennungsinformationen, wobei im Speicher (300) verschiedene Daten hinterlegt sind, die entsprechend den Kennungsinformationen dem medizinischen Sensor (100) zugeordnet sind,
wobei der medizinische Sensor (100) ferner Folgendes umfasst: einen Konfigurationsabschnitt (130), der mit dem ersten Kommunikationsabschnitt (120) verbunden ist, um die Initialisierung und die Parameterfestlegung des medizinischen Sensors (100) entsprechend den Initialisierungsparametern automatisch abzuschließen, und
wobei die Bedienvorrichtung (200) ferner Folgendes umfasst: einen zweiten Kommunikationsabschnitt (230) zum Senden der Initialisierungsparameter an den medizinischen Sensor (100) und zum Anweisen des medizinischen Sensors (100), anhand der Initialisierungsparameter automatisch eine Initialisierung und eine Parameterfestlegung durchzuführen,
wobei der Verarbeitungsabschnitt (220) ferner dazu eingerichtet ist, dem medizinischen Sensor (100) zugeordnete Diagnose- und Behandlungsinformationen anhand der Kennungsinformationen aus dem Speicher (300) zu erhalten, wobei die Diagnose- und Behandlungsinformationen den Namen des Krankenhauses und/oder den Namen der Abteilung und/oder Ausweisdaten von medizinischem und Pflegepersonal enthalten, und
wobei der zweite Kommunikationsabschnitt (230) ferner dazu eingerichtet ist, Erfassungsergebnisse des medizinischen Sensors (100) von dem medizinischen Sensor (100) zu empfangen, und
wobei der Verarbeitungsabschnitt (220) ferner dazu eingerichtet ist, die Erfassungsergebnisse als Reaktion auf die von dem zweiten Kommunikationsabschnitt (230) empfangenen Erfassungsergebnisse an eine Diagnose- und Behandlungsinformationen zugeordnete Benutzerstation zu senden.

2. Medizinisches System nach Anspruch 1, bei dem
der Konfigurationsabschnitt (130) ferner dazu eingerichtet ist, eine Startmeldung zu erzeugen, die angibt, dass der medizinische Sensor (100) normal zu funktionieren beginnt, wenn die Initialisierung und die Parameterfestlegung abgeschlossen sind, und
der erste Kommunikationsabschnitt (120) ferner dazu eingerichtet ist, die Startmeldung an die Bedienvorrichtung (200) zu senden.

3. Medizinisches System nach Anspruch 1, bei dem
der erste Kommunikationsabschnitt (120) ferner dazu eingerichtet ist, Erfassungsergebnisse des medizinischen Sensors (100) an die Bedienvorrichtung (200) zu senden.

4. Medizinisches System nach Anspruch 1, bei dem der Verarbeitungsabschnitt (220) ferner dazu eingerichtet ist,
anhand der Kennungsinformationen einen Betriebsdatensatz des medizinischen Sensors (100) aus dem Speicher (300) zu erhalten, wobei der Betriebsdatensatz die Nutzungsanzahl und/oder die aufgelaufene Betriebsdauer und/oder die zulässige Nutzungsanzahl und/oder die zulässige Betriebsdauer und/oder den zulässigen Verwendungsbereich und/oder die Lebensdauer des medizinischen Sensors (100) enthält,
anhand des Betriebsdatensatzes zu ermitteln, ob der medizinische Sensor (100) verwendet werden darf oder nicht, und
dann, wenn festgestellt wird, dass der medizinische Sensor (100) nicht verwendet werden darf, eine Deaktivierungsmeldung zu erzeugen, die angibt, dass die Verwendung des medizinischen Sensors (100) unzulässig ist.

5. Medizinisches System nach Anspruch 4, bei dem
der zweite Kommunikationsabschnitt (230) ferner dazu eingerichtet ist, vom medizinischen Sensor (100) eine Startmeldung zu empfangen, die angibt, dass der medizinische Sensor (100) normal zu funktionieren beginnt, und
der Verarbeitungsabschnitt (220) ferner dazu eingerichtet ist, den im Speicher (300) hinterlegten Betriebsdatensatz des medizinischen Sensors (100) als Reaktion auf die vom zweiten Kommunikationsabschnitt (230) empfangene Startmeldung zu aktualisieren.

6. Medizinisches System nach Anspruch 5, bei dem der Verarbeitungsabschnitt (220) ferner dazu eingerichtet ist,
die Zeit zu akkumulieren, die seit dem Senden des Initialisierungsparameters verstrichen ist, und
dann, wenn die akkumulierte Zeit eine vorbestimmte Zeitspanne überschreitet, der zweite Kommunikationsabschnitt (230) jedoch immer noch nicht die Startmeldung empfängt, eine Fehlermeldung zu erzeugen, die den Ausfall des medizinischen Sensors (100) angibt.

7. Medizinisches System nach einem der Ansprüche 1 bis 6, bei dem der Speicher (300) eine in die Bedienvorrichtung (200) eingebaute interne Datenspeichervorrichtung und/oder eine an der Bedienvorrichtung (200) anbringbare tragbare Datenspeichervorrichtung und/oder einen entfernten Datenspeicherserver aufweist, der in der Lage ist, mit der Bedienvorrichtung (200) zu kommunizieren.

8. Verfahren zur Verwendung eines medizinischen Sensors eines medizinischen Systems nach einem der vorhergehenden Ansprüche, bei dem:
eine Kennung des medizinischen Sensors abgetastet wird, um Kennungsinformationen zu erhalten, die in dem medizinischen Sensor enthalten und durch die Kennung angegeben sind (S301),
anhand der Kennungsinformationen Initialisierungsparameter des medizinischen Sensors aus einem Speicher erhalten werden, wobei im Speicher verschiedene Daten hinterlegt sind, die dem medizinischen Sensor entsprechend den Kennungsinformationen zugeordnet sind (S302),
veranlasst wird, dass die Initialisierungsparameter an den medizinischen Sensor gesendet werden (S303), und
der medizinische Sensor angewiesen wird, anhand der Initialisierungsparameter automatisch eine Initialisierung und eine Parameterfestlegung durchzuführen, und
nach Erhalt der durch die Kennung angegebenen Kennungsinformationen des medizinischen Sensors ferner anhand der Kennungsinformationen dem medizinischen Sensor zugeordnete Diagnose- und Behandlungsinformationen aus dem Speicher erhalten werden (S601), wobei die Diagnose- und Behandlungsinformationen den Namen des Krankenhauses und/oder den Namen der Abteilung und/oder Ausweisdaten von medizinischem und Pflegepersonal enthalten, und
nach dem Senden der Initialisierungsparameter an den medizinischen Sensor ferner von dem medizinischen Sensor Erfassungsergebnisse des medizinischen Sensors empfangen werden (S602), und
die Erfassungsergebnisse als Reaktion auf den Empfang der Erfassungsergebnisse an eine den Diagnose- und Behandlungsinformationen zugeordnete Benutzerstation gesendet werden (S603).

9. Verfahren nach Anspruch 8, bei dem ferner vor dem Senden der Initialisierungsparameter an den medizinischen Sensor:
anhand der Kennungsinformationen ein Betriebsdatensatz des medizinischen Sensors aus dem Speicher erhalten wird (S401), wobei der Betriebsdatensatz die Nutzungsanzahl und/oder die aufgelaufene Betriebsdauer und/oder die zulässige Nutzungsanzahl und/oder die zulässige Betriebsdauer und/oder den zulässigen Verwendungsbereich und/oder die Lebensdauer des medizinischen Sensors enthält,
anhand des Betriebsdatensatzes ermittelt wird, ob der medizinische Sensor verwendet werden darf oder nicht (S402), und
dann, wenn festgestellt wird, dass der medizinische Sensor nicht verwendet werden darf, eine Deaktivierungsmeldung erzeugt wird, die angibt, dass die Verwendung des medizinischen Sensors unzulässig ist (S403).

10. Verfahren nach Anspruch 9, bei dem ferner nach dem Senden der Initialisierungsparameter an den medizinischen Sensor:
eine Startmeldung vom medizinischen Sensor empfangen wird, die angibt, dass der medizinische Sensor normal zu funktionieren beginnt (S502), und
der im Speicher hinterlegte Betriebsdatensatz des medizinischen Sensors als Reaktion auf die empfangene Startmeldung aktualisiert wird (S503).

11. Verfahren nach Anspruch 10, bei dem ferner nach dem Senden der Initialisierungsparameter an den medizinischen Sensor:
die Zeit akkumuliert wird, die seit dem Senden des Initialisierungsparameters verstrichen ist (S501), und
dann, wenn die akkumulierte Zeit eine vorbestimmte Zeitspanne überschreitet, die Startmeldung jedoch immer noch nicht empfangen wird, eine Fehlermeldung erzeugt wird, die den Ausfall des medizinischen Sensors angibt (S504).

## Revendications

1. Système médical, comprenant un capteur médical (100) et un dispositif d'actionnement (200),
le capteur médical (100) comprenant :
une identification (110) qui indique des informations d'identification du capteur médical (100) et qui est apte à être identifiée par le dispositif d'actionnement (200), l'identification (110) comprenant un code bidimensionnel et/ou un code-barres et/ou une apparence spécifique et/ou une étiquette RFID ; et
une première section de communication (120) pour la réception de paramètres d'initialisation correspondant aux informations d'identification identifiées du dispositif d'actionnement (200) ; et
le dispositif d'actionnement (200) comprenant :
une section de balayage (210) pour balayer l'identification (110) inclue dans le capteur médical (100) pour obtenir les informations d'identification du capteur médical (100) indiquées par l'identification (110), la section de balayage (210) comprenant un lecteur de code bidimensionnel et/ou un lecteur de code-barres et/ou un lecteur d'étiquette RFID ;
une section de traitement (220) pour obtenir des paramètres d'initialisation du capteur médical (100) provenant d'une mémoire (300) sur la base des informations d'identification, la mémoire (300) stockant différentes données associées au capteur médical (100) selon les informations d'identification ;
le capteur médical (100) comprenant en outre : une section de configuration (130) qui est connectée à la première section de communication (120) pour automatiquement compléter l'initialisation et la détermination de paramètres du capteur médical (100) selon les paramètres d'initialisation ; et
le dispositif d'actionnement (200) comprenant en outre : une deuxième section de communication (230) pour envoyer les paramètres d'initialisation au capteur médical (100) et donner des instructions au capteur médical (100) pour que celui-ci réalise automatiquement l'initialisation et la détermination de paramètres sur la base des paramètres d'initialisation ;
la section de traitement (220) étant en outre réalisée de manière à obtenir des informations de diagnostic et de traitement associées au capteur médical (100) provenant de la mémoire (300) sur la base des informations d'identification, les informations de diagnostic et de traitement comprenant un nom d'hôpital et/ou un nom de service et/ou une identification de personnel médical et de personnel soignant, et
la deuxième section de communication (230) étant en outre réalisée de manière à recevoir des résultats de détection du capteur médical (100) du capteur médical (100) ; et
la section de traitement (220) étant en outre réalisée de manière à envoyer les résultats de détection à un terminal d'utilisateur associé aux informations de diagnostic et de traitement en réponse aux résultats de détection reçues par la deuxième section de communication (230).

2. Système médical selon la revendication 1, dans lequel
la section de configuration (130) est en outre réalisée de manière à générer un message de démarrage indiquant que le capteur médical (100) commence à travailler normalement lorsque l'initialisation et la détermination de paramètres sont terminées ; et
la première section de communication (120) est en outre réalisée de manière à envoyer le message de démarrage au dispositif d'actionnement (200).

3. Système médical selon la revendication 1, dans lequel
la première section de communication (120) est en outre réalisée de manière à envoyer des résultats de détection du capteur médical (100) au dispositif d'actionnement (200).

4. Système médical selon la revendication 1, dans lequel la section de traitement (220) est en outre réalisée de manière
à obtenir, sur la base des informations d'identification, un ensemble de données de service du capteur médical (100) provenant de la mémoire (300), l'ensemble de données de service comprenant un nombre d'utilisations et/ou une période de service accumulée et/ou un nombre d'utilisations autorisé et/ou une période de service autorisée et/ou un domaine d'utilisation autorisé et/ou une durée de service du capteur médical (100) ;
à déterminer, sur la base de l'ensemble de données de service, si l'utilisation du capteur médical (100) est autorisée ou non ; et
à générer un message de désactivation indiquant que l'utilisation du capteur médical (100) n'est pas autorisée lorsqu'il est déterminé que l'utilisation du capteur médical (100) n'est pas autorisée.

5. Système médical selon la revendication 4, dans lequel
la deuxième section de communication (230) est en outre réalisée de manière à recevoir un message de démarrage indiquant que le capteur médical (100) commence à travailler normalement et provenant du capteur médical (100) ; et
la section de traitement (220) est en outre réalisée de manière à mettre à jour l'ensemble de données de service du capteur médical (100) stocké dans la mémoire (300) en réponse au message de démarrage reçu par la deuxième section de communication (230).

6. Système médical selon la revendication 5, dans lequel la section de traitement (220) est en outre réalisée de manière
à accumuler le temps écoulé à partir de l'envoi du paramètre d'initialisation ; et
à générer un message d'erreur indiquant une défaillance du capteur médical (100) lorsque le temps accumulé dépasse une période de temps prédéterminée mais la deuxième section de communication (230) n'a pas encore reçu le message de démarrage.

7. Système médical selon l'une des revendications 1 à 6, dans lequel la mémoire (300) présente un dispositif de stockage de données interne installé dans le dispositif d'actionnement (200) et/ou un dispositif de stockage de données portable apte à être rattaché au dispositif d'actionnement (200) et/ou un serveur de stockage de données à distance apte à communiquer avec le dispositif d'actionnement (200).

8. Procédé d'utilisation d'un capteur médical d'un système médical selon l'une des revendications précédentes, dans lequel :
une identification du capteur médical est balayée pour obtenir des informations d'identification inclues dans le capteur médical et indiquées par l'identification (S301) ;
des paramètres d'initialisation du capteur médical provenant d'une mémoire sont obtenus sur la base des informations d'identification, la mémoire stockant différentes données associées au capteur médical selon les informations d'identification (S302) ;
les paramètres d'initialisation sont amenés à être envoyés au capteur médical (S303) ; et
des instructions sont données au capteur médical pour que celui-ci réalise automatiquement l'initialisation et la détermination de paramètres sur la base des paramètres d'initialisation ; et
après l'obtention des informations d'identification du capteur médical indiquées par l'identification, des informations de diagnostic et de traitement associées au capteur médical et provenant de la mémoire sont en outre obtenues sur la base des informations d'identification (S601), les informations de diagnostic et de traitement comprenant un nom d'hôpital et/ou un nom de service et/ou une identification de personnel médical et de personnel soignant, et
après l'envoi des paramètres d'initialisation au capteur médical, des résultats de détection du capteur médical sont en outre reçus du capteur médical (S602) ; et
les résultats de détection sont envoyés à un terminal d'utilisateur associé aux informations de diagnostic et de traitement en réponse à la réception des résultats de détection (S603).

9. Procédé selon la revendication 8, dans lequel, avant l'envoi des paramètres d'initialisation au capteur médical :
un ensemble de données de service du capteur médical provenant de la mémoire est obtenu sur la base des informations d'identification (S401), l'ensemble de données de service comprenant un nombre d'utilisations et/ou une période de service accumulée et/ou un nombre d'utilisations autorisé et/ou une période de service autorisée et/ou un domaine d'utilisation autorisé et/ou une durée de service du capteur médical ;
il est déterminé si l'utilisation du capteur médical est autorisée ou non sur la base de l'ensemble de données de service (S402) ; et
un message de désactivation indiquant que l'utilisation du capteur médical n'est pas autorisée est généré lorsqu'il est déterminé que l'utilisation du capteur médical n'est pas autorisée (S403).

10. Procédé selon la revendication 9, dans lequel, après l'envoi des paramètres d'initialisation au capteur médical :
un message de démarrage est reçu du capteur médical, indiquant que le capteur médical commence à travailler normalement (S502) ; et
l'ensemble de données de service du capteur médical stocké dans la mémoire est mis à jour en réponse au message de démarrage reçu (S503).

11. Procédé selon la revendication 10, dans lequel, après l'envoi des paramètres d'initialisation au capteur médical :
le temps écoulé depuis l'envoi du paramètre d'initialisation est en outre accumulé (S501) ; et
un message d'erreur indiquant une défaillance du capteur médical est généré lorsque le temps accumulé dépasse une période de temps prédéterminée mais le message de démarrage n'a pas encore été reçu (S504).
